# EUROPEAN PATENT SPECIFICATION

(11) **EP 0 339 092 B1**
(45) Date of publication and mention of the grant of the patent: **04.06.1997**
(21) Application number: 88900130.1
(22) Date of filing: 22.12.1987
(51) Int. Cl.: A61B 10/00, A61D 1/08

(54) **APPARATUS FOR DETECTING A FERTILE PERIOD OF MAMMALS**
GERÄT ZUR ANZEIGE DER FRUCHTBAREN PERIODE BEI SÄUGETIEREN
DETECTEUR DE PERIODE DE FERTILITE CHEZ LES MAMMIFERES

(30) Priority: 22.12.1986 JP 305807/86; 05.06.1987 JP 141272/87
(43) Date of publication of application: 02.11.1989
(73) Proprietor: KABUSHIKI KAISHA SEMEX JAPAN, Shizuoka 433 (JP)
(72) Inventor: MATSUURA, Masayuki Kabushiki Kaisha Semex Japan, Hamamatsu-shi Shizuoka 433 (JP)
(74) Representative: Grünecker, Kinkeldey, Stockmair & Schwanhäusser Anwaltssozietät
(86) International application number: JP8701011
(87) International publication number: WO8804538

(56) References cited:
- JP-A-61 113 437
- US-A- 3 844 276
- US-A- 4 498 481

## Description

### TECHNICAL FIELD

The present invention relates to an electrical fertility detecting apparatus for detecting the fertility of mammals including men, as set forth in the preamble of the claim. An apparatus of this kind is known from US-A-4 498 481.

### BACKGROUND ART

From afore-mentioned US-A-4,498,481 an oestrus cycle monitoring system for detecting oestrus in female mammals and human females is known by which the electrical resistance of mucus in the vaginal cavitiy is measured. The system comprises a hand-held electrical package including readout means for temperature as well as for conductivity of the mucus and comprises a longitudinal probe supported by a stainless steel elongated rod for positioning in the vagina. The probe includes two gold-plated twin-helix electrodes supported in molded plastic about the circumference of the probe, and an objective lens with a diffuser lens surrounding same for transmitting light for viewing of the vagina and cervix. The utilization of a fiber-optic scope, electrical resistance reading of the mucus and temperature of the vagina provide for determining the stage of oestrus.

Other fertility or ovulation detecting apparatus which detect the fertility or ovulation of mammals on the basis of the equivalent DC resistance on the mammal's vaginal mucous membrane have been proposed, for example, in Japanese Patent Laid-open (Kokai) Nos. 60-188142 through 188148, 60-190942, 60-190943, 60-190944, 60-220052, 60-227746, 61-90651 through 90655, 61-137543, 61-137544, 61-187545, 61-137554 and 61-217157. These apparatus detect the sodium ion concentration on the mammal's vaginal mucous membrane on the basis of the equivalent DC resistance which varies in inverse proportion to the sodium ion concentration.

Each foregoing known ovulation predicting apparatus or fertility detecting apparatus is designed on the basis of an empirical rule that the equivalent DC resistance on the vaginal mucous membrane increases to a maximum level just before ovulation, and is provided with lamps respectively corresponding to the maximum level, the minimum level and a plurality of levels between the maximum and minimum levels of equivalent DC resistance. Each lamp lights up when the equivalent DC resistance on the vaginal mucous membrane coincides with the corresponding level of equivalent DC resistance.

With hogs, for instance, it is generally accepted that insemination is achieved successfully at a maximum conception ratio when the chilled semen or the live semen is introduced into the uterus five to seven hours before ovulation and when the frozen semen is introduced into the uterus two hours before ovulation.

Accordingly, it is most desirable to light up the lamb corresponding to a detected equivalent DC resistance on the vaginal mucous membrane when the detected equivalent DC resistance corresponds to an equivalent DC resistance in a period five to seven hours before or two hours before ovulation.

However, the maximum equivalent DC resistance on the vaginal mucous membrane, which is reached just before ovulation, and the minimum equivalent DC resistance, which is reached in an unfertile period, namely, a period in which ovulation does not occur, have not been known so far. Accordingly, the lamp corresponding to an equivalent DC resistance representing vaginal conditions suitable for insemination of the known ovulation predicting apparatus or fertility detecting apparatus does not necessarily indicate an accurate period suitable for depositing the semen in the uterus.

Therefore, artificial insemination has not successfully been achieved at an conception ratio of 100% even if artificial insemination was carried out in an appropriate inseminating period determined by the ovulation predicting apparatus or the fertility detecting apparatus. Furthermore, it has been impossible to use the fertility detecting apparatus for man for the purpose of birth control, because the minimum DC resistance on the vaginal mucous membrane, namely, a criterion for zero point setting, is indefinite.

With the foregoing in view, it is an object of the present invention to provide a fertility detecting apparatus for mammals, capable of exactly determining the fertile state of mammals, capable of setting an accurate zero point, and capable of ensuring substantialy 100% successful insemination.

It is another object of the present invention to provide a fertility detecting apparatus applicable to birth control.

### DISCLOSURE OF THE INVENTION

The present invention has been made on the basis of findings that the sodium ion concentration on the vaginal mucous membrane reaches a minimum sodium ion concentration approximately corresponding to that of water during ovulation and reaches a maximum sodium ion concentration approximately corresponding to the sodium ion concentration of the blood of the mammal during an unfertile period, and that, although it has been supposed that the sodium ion concentration varies in inverse proportion to the equivalent DC resistance on the vaginal mucous membrane, the sodium ion concentration varies in inverse proportion to the equivalent impedance on the vaginal mucous membrane.

To achieve the foregoing object, the present invention provides a fertility detecting apparatus for mammals set forth in the claim.

The sodium ion concentration detecting means comprises: a detecting unit which is inserted in the vagina of the mammal; a plurality of electrodes arranged on the detecting unit so as to be in contact with the vaginal mucous membrane when the detecting unit is inserted in the vagina of the mammal; a voltage generating means for applying a voltage across the plurality of electrodes; and impedance detecting means for detecting the impedance between the electrodes. The indicating means indicates an unfertile period where a detected equivalent impedance of the vaginal mucous membrane detected by the detecting unit coincides with an equivalent impedance representing an unfertile period, which is determined on the basis of a maximum equivalent impedance substantially equal to that of water which is reached just before ovulation, and a minimum equivalent impedance substantially equal to that of the mammal's blood which is reached in an unfertile period.

The present invention is based on a fact that the sodium ion concentration on the vaginal mucous membrane is reaches a minimum sodium ion concentration substantially the same as that of water just before ovulation, and the indicating means indicates a period suitable for introducing the semen into the uterus before ovulation on the basis of the minimum sodium ion concentration. Accordingly, a period of ovulation and a fertile period determined on the basis of the moment of ovulation can accurately be detected.

Furthermore, since the indicating means of the present invention is designed so as to indicate an unfertile period on the basis of a fact that the sodium ion concentration on the mammal's vaginal mucous membrane reaches a maximum sodium ion concentration, which is substantially equal to the sodium ion concentration of the blood of the mammal, during an unfertile period, the zero point of the indicating means can accurately be set. Accordingly, the unfertilizability detecting apparatus of the present invention is applicable to birth control.

### BRIEF DESCRIPTION OF THE DRAWINGS

Figure 1 is a front elevation of a fertility detecting apparatus for mammals, in a preferred embodiment, according to the present invention;
Figure 2 is an electric circuit diagram of the electrical construction of the fertility detecting apparatus of Fig. 1;

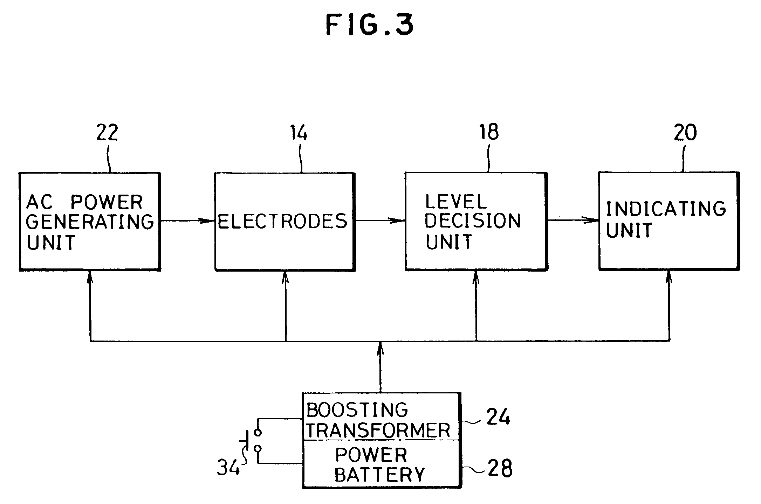

Figure 3 is a block diagram of the fertility detecting apparatus of Fig. 1;
Figure 4 is a graph showing the variation of the impedance on the hog's vaginal mucous membrane with time; and
Figure 5 is a front elevation of a fertility detecting apparatus, in another embodiment, according to the present invention.

### BEST MODE FOR CARRYING OUT THE INVENTION

The preferred embodiments of the present invention will be described hereinafter with reference to the accompanying drawings.

Referring to the drawings, a fertility detecting apparatus in a first embodiment according to the present invention comprises: a bar-shaped detecting unit 12; an impedance detecting unit 10 comprising a pair of electrodes 14 arranged on the extremity 12A of the detecting unit 12 so as to be in contact with the mammal's vaginal mucous membrane, a voltage generating unit 16 for applying an ac voltage across the electrodes 14, and a level decision unit 18 for detecting the impedance between the electrodes 14, and an indicating unit 20 which indicates an appropriate semen deposition period before ovulation, and an unfertile period in which ovulation does not occur, on the basis of a maximum equivalent impedance on the vaginal mucous membrane substantially equal to that of water, which is reached just before ovulation, and a minimum equivalent impedance on the mammal's vaginal mucous membrane substantially equal to that of the blood of the mammal, which is existent in the unfertile period.

The voltage generating unit 16 has an ac power generator 22, a boosting transformer 24, and a voltage comparator 26.

The ac power generating unit 22 includes an astable multivibrator 22A and a positive-negative pulse amplifier 22B.

The astable multivibrator 22A comprises, as principal components, transistors TR1 and TR2, resistors R1 to R4, and capacitors C1 and C2. The positive-negative amplifier 22B has transistors TR3 to TR7 and resistors R5 to R16 for amplifying pulses provided by the astable multivibrator 22A.

The boosting transformer 24 comprises an inverter circuit for raising the output voltage of a power battery 28 to a necessary voltage, comprising amplifying transistors TR8 and TR9, capacitors C4 to C7 to apply the output voltage of the power battery 28 to the transistors TR8 and TR9 for raising the output voltage of the power battery 28, resistors R25 to R27, coils L1 and L2, a diode D2, and a Zener diodes ZD1 and ZD2. The respective positive terminals of the diode D2 and the Zener diode ZD1 are connected to the resistor R10 of the ac generating unit 22 and to the resistor R17 of the level decision unit 30 to receive an increased voltage and a current.

The level decision unit 18 has a plurality of voltage comparators for detecting the impedance level of an impedance equivalent circuit 18A of an objective (the vaginal mucous membrane) by means of an ac voltage applied across the electrodes 14. The indicating unit 20 has light emitting diodes DP1 to DP5 respectively corresponding to impedance levels determined by the level decision unit 18 to indicate the corresponding impedance levels.

The level decision unit 18 comprises: resistors R17 to R23 for dividing a voltage applied thereto by the boosting transformer 24; operational amplifiers OP1 to OP6 for comparing the divided voltages with a voltage signal developed across the electrodes 14 and provided through the diode D1; and exclusive OR circuits EOR1 to EOR5 to generate light emitting diode driving signals for driving the light emitting diodes DP1 to DP5 of the indicating unit 20 on the basis of the output signals of the operational amplifiers OP1 to OP6. The level decision unit 18 decides the impedance level of the impedance equivalent circuit 18A on the basis of the voltage signal provided through the diode D1. Elements including a capacitor C3 and a resistor R24 are connected to the positive terminal of the diode D1 to stabilize the input voltage signal given to the operational amplifiers OP1 to OP6.

The voltage comparator 26 is used for checking the output voltage of the power battery 28. An operating switch 32 is closed to actuate the voltage comparator 26.

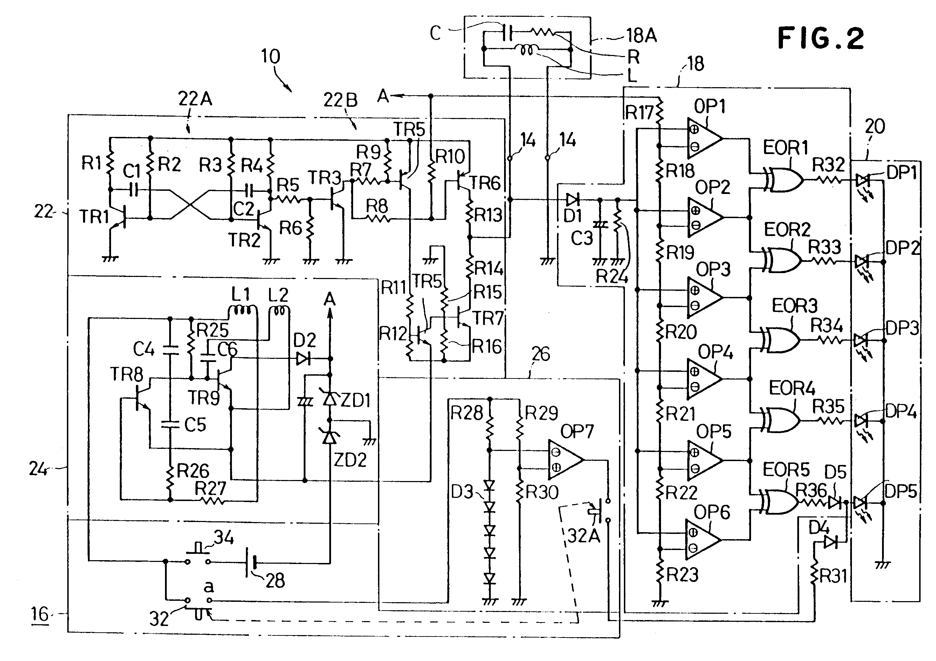

In Fig. 2, indicated at 34 is an operating switch for actuating the level decision unit 18.

The voltage comparator 26 has resistors R28 to R30, a series arrangement D3 of diodes, resistors R28 to R30, and an operational amplifier OP7 for comparing the voltage divided by the series arrangement D3 of diodes. A decision signal representing the result of comparative check is give through a contact 32A interlocked with the operating switch 32 for simultaneous operation to the light emitting diode DP5 to drive the light emitting diode DP5 according to the decision signal. The output signal of the operational amplifier OP7 is given through a resistor 31 and a diode D4 to the light emitting diode D5. A diode D5 is provided between the diode D4 and the exclusive OR circuit EOR5 to prevent the backward application of the decision signal t the exclusive OR circuit EOR5. Voltage dividing resitors R32 to R36 are connected respectively to the output terminals of the exclusive OR circuits EOR1 to EOR5 to divide the driving signals applied respectively to the light emitting diodes DP1 to DP5.

The sum of the resistances of the voltage dividing resistors R18 to R23 is slightly smaller than the impedance of water. The hog's normal body temperature, for instance, is 38.5° C. Accordingly, the sum of the resistances of the resistors R18 to R23 is on the order of 2.14 kΩ at 38.5° C, which is slightly smaller than the equivalent impedance of water across the electrodes 14 (hereinafter an impedance across the electrodes 14 will be referred to simply as "impedance").

Ordinarily, human beings drink city water and well water, which are different from each other in equivalent impedance. Accordingly, the sum of the resistances of the voltage dividing resistors R18 to R23 is decided roughly. Concretely, the sum of the resitances of the resitors R18 to R23 is decided so that the light emitting diode DP1 lights up when the electrodes 14 are immersed in city water.

The resistance of the voltage dividing resistor R23 is substantially the same as the equivalent impedance of the blood of the objective mammal, which is, for example, a value on the order of 0.78 kΩ for the hog. Concretely, the resistance of the voltage dividing resistor R23 is decided so that the light emitting diode DP5 lights up when the electrodes 14 are immersed in the blood.

The sum of the resistances of the voltage dividing resistors R19 to R23 is substantially the same as the equivalent impedance on the mammal's vaginal mucous membrane at an appropriate period for depositing the chilled semen in the uterus, which is, for example, a value on the order of 1.88 kΩ for the hog.

The sum of the resistances voltage dividing resistors R20 to R23 is substantially the same as the equivalent impedance on the mammal's vaginal mucous membrane at an appropriate period for depositing the frozen semen in the uterus of the mammal, which is, for example a value on the order of 1.644 kΩ for the hog.

The sum of the resistances of the voltage dividing resistors R21 to R23 is substantially the same as the maximum equivalent impedance on the mammal's vaginal mucous membrane during an unfertile period in which ovulation does not occur, which is, for example, 1.00 kΩ for the hog.

The sum of the resitances of the voltage dividing resistors R22 and R23 is substantially the same as the lower half of the equivalent impedance on the mammal's vaginal mucous membrane during an unfertile period of the mammal in which ovulation does not occur, which is, for example, a value on the order of 0.867 kΩ for the hog.

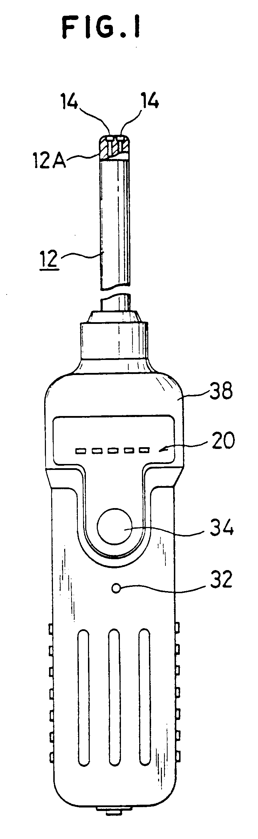

As shown in Fig. 1, the bar-shaped detecting unit 12 mounted with the electrodes 14 is attached to a body 38 containing the detecting circuit and the power battery.

The light emitting diodes DP1 to DP5 of the indicating unit 20, and the operating switches 32 and 34 are arranged on the surface of the body 38.

Process of detecting the fertile period of the mammal by the fertility detecting apparatus embodying the present invention will be described hereinafter.

First, the bar-shaped detecting unit 12 is inserted in the vagina of the mammal.

Then, the electrodes 14 provided on the extremity of the bar-shaped detecting unit 12 is pressed against the vaginal mucous membrane, and then the operating switch 34 is closed while the electrodes 14 are pressed against the vaginal mucous membrane.

Then, a voltage proportionate to the value of the impedance equivalent circuit 18A provided between the electrodes 14 is applied through the diode D1 to the respective non inverting terminals of the operational amplifiers OP1 to OP6. On the other hand, divided voltages divided by the resistors R17 to R23 are applied respectively to the inverting input terminals of the operational amplifiers OP1 to OP6. The results of comparison of the divided voltages applied respectively to the operational amplifiers OP1 to OP6 with the voltage proportionate to the impedance equivalent circuit 18A are given to the exclusive OR circuits EOR1 to EOR5. The divided voltages applied respectively to the inverting terminals of the operational amplifiers OP1 to OP6 increase stepwise according to the resistances of the resistors R23 to R17 in the order of the operational amplifiers OP6 to OP1.

The exclusive OR circuits EOR1 to EOR5 perform the function of the exclusive or respectively for the outputs of the adjacent pairs of the operational amplifiers OP1 to OP6. Consequently, one of the light emitting diodes DP1 to DP5 depending on the value of the impedance equivalent circuit 18A.

As mentioned above, the sum of the resistances of the voltage dividing resistors R18 to R23 is substantially equal to the equivalent impedance of water and the sum of the resistances of the voltage dividing resistors R19 to R23 is equal to the equivalent impedance on the vaginal mucous membrane in a condition suitable for introducing the chilled semen into the uterus. Accordingly, the light emitting diode DP1 lights up when the vaginal mucous membrane is in a condition suitable for introducing the frozen semen into the uterus. Similarly, the light emitting diode DP2 lights up when the vaginal mucous membrane is in a condition suitable for introducing the chilled semen into the uterus.

When the impedance between the electrodes 14 is equal to the equivalent impedance of water, the output signals of the operational amplifiers OP1 and OP2 are applied to the exclusive OR circuits EOR1, thereby the light emitting diode DP1 is turned off.

Accordingly, the electrodes 14 are immersed in water to check the fertility indicating apparatus.

Since the sum of the resistances of the voltage dividing resistors R21 to R23 is equal to the maximum impedance of the impedance equivalent circuit 18A corresponding to that on the vaginal mucous membrane in the unfertile period where no ovulation occurs, the mammal is not fertile when the light emitting diode DP4 or DP5 lights up.

Since the light emitting diode DP4 represents a high-level impedance in the unfertile period and the light emitting diode DP5 represents a low-level impedance in the unfertile period, it is possible to decide if ovulation is approaching and if ovulation has already occurred from the condition of the light emitting diodes DP4 and DP5 when the mammal is in the matting season. The light emitting diode DP3 indicates an intermediate period between the fertile period and the unfertile period, namely, a so-called gray zone in which the fertility of the mammal is indefinite.

Since the resistance of the resistor R23 is slightly greater than the impedance of the objective mammal's blood, the light emitting diode DP5 is turned off when the electrodes 14 are immersed in the objective mammal's blood.

Thus, the zero level of the fertility detecting apparatus can be checked by immersing the electrodes 14 in the blood.

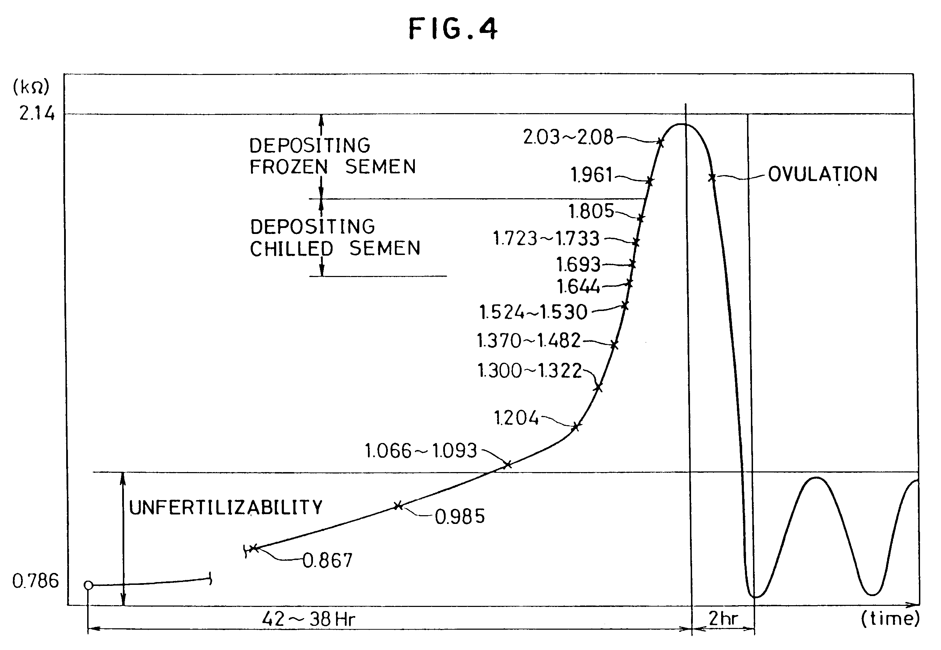

As is obvious from Fig. 4 showing a measured curve of the equivalent impedance on the hog's vaginal mucous membrane, the equivalent impedance on the vaginal mucous membrane varies periodically with time between the zero point (the equivalent impedance of the blood) and 1 kΩ along a stable curve during a period in which no ovulation occurs, starts increasing from a moment forty-two to thirty-eight hours before ovulation and approaches the equivalent impedance of water infinitely, becomes substantially equal to the equivalent impedance of water just before ovulation, and then decreases sharply to a minimum in two to three hours after reaching a maximum.

Ordinary the chilled semen is introduced into the vagina about five to seven hours before ovulation for the artificial insemination of the hog. Since the relation between the equivalent impedance on the hog's vaginal mucous membrane and time is constant, appropriate semen introducing timing is achieved by constructing the fertility detecting apparatus so that the light emitting diode DP2 turns on seven to five hours before ovulation.

When frozen semen is used, successful artificial insemination can be achieved at a maximum conception rate by depositing the frozen semen two hours before ovulation, because a capsule (antigen, protoplasm and tunic) covering a spermatozoon has been removed to capacitate the spermatozoon for insemination and hence the frozen semen is readily functioning for fertilization as compared with the chilled or live semen. Accordingly, the frozen semen is deposited when the light emitting diode DP1 lights up. It is impossible to achieve fertilization when the frozen semen is deposited when either one of the light emitting diodes DP2 to DP5 is on.

Although the fertility detecting apparatus in this embodiment detects the level of the impedance by the level decision unit 18, the present invention is not limited thereto in its application; any suitable impedance detector may be used provided that the impedance detector is able to detect the impedance or able to decide the level of the impedance among a plurality of levels.

Although the indicating unit 20 of the foregoing embodiment has the plurality of light emitting diodes DP1 to DP5, the present invention is not limited thereto in its application; any suitable indicating means capable of indicating values betweeen the maximum impedance and the minimum impedance of the impedance equivalent circuit 18A of the vaginal mucous membrane may be employed.

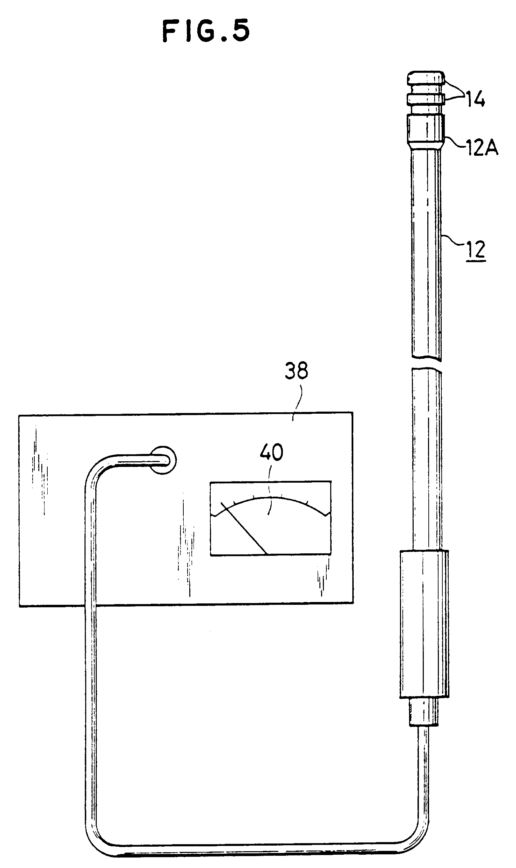

Accordingly, the indicating unit 20 may be an ordinary impedance meter 36 as shown in Fig. 5. In a second embodiment employing such an impedance meter, equivalent impedances between the maximum equivalent impedance corresponding to that of water and the minimum equivalent impedance corresponding to the blood, divided into suitable levels can be indicated on the impedance meter, and hence the level decision unit 18 can be substituted by a single resistor. The second embodiment is capable of detecting the ovulation period and unfertile period more accurately than the first embodiment.

The number of the light emitting diodes need not be limited to five, but may be two, three or not less than six.

Furthermore, although the fertility detecting apparatus in the foregoing embodiment detect the ovulation period on the basis of the equivalent impedance on the vaginal mucous membrane, the present invention is not limited thereto in its application; any suitable detecting means capable of directly or indirectly detecting the sodium ion concentration on the vaginal mucous membrane may be employed.

Accordingly, the sodium ion concentration on the vaginal mucous membrane may be measured instead of the impedance.

Still further, although the present invention has been described as applied to the detection of the fertility of the hog, naturally, the present invention is applicable to the detection of the fertility of other mammals including men.

### CAPABILITY OF EXPLOITATION IN INDUSTRY

The fertility detecting apparatus thus constructed according to the present invention is capable of exactly detecting the fertilizable period and unfertile period of mammals.

The results of experimental application of the fertility detecting apparatus of the present invention to artificial insemination of hogs and cows showed that the percentage of successful insemination is 100% excluding abnormal cases such as hogs and cows suffering from endometritis, when the level decision unit (the impedance detector) is set for a maximum value substantially equal to the equivalent impedance of water and for a minimum value substantially eqaul to the equivalent impedance of the blood of the objective mammal.

## Claims

1. An apparatus for detecting an optimal fertility for mammals, comprising:
means (10) for measuring sodium ion concentration for detecting a measured sodium ion concentration on a mammal's vaginal mucous membrane, said measuring means including:
a detecting unit (12) to be inserted into a mammal's vagina;
a plurality of electrodes (14) so provided on said detecting unit (12) as to be in contact with the vaginal mucous membrane of said mammal when said detecting unit (12) is inserted into the vagina of said mammal;
a voltage generating means (16) for applying a voltage across said plurality of electrodes (14); and
an impedance detector (18) for detecting the impedance between said plurality of electrodes (14);
characterized by further comprising
means (OP1-OP6) for comparing the equivalent impedance value detected by said impedance detector (18) with respect to a maximum value to determine and indicate a time period, before ovulation, optimum for injecting spermatozoon into the vagina, and for comparing said detected value with respect to a minimum value to determine and indicating an unfertile time period in non-ovulation, where the maximum value is set to represent a time period immediately before ovulation when the detected equivalent impedance on the vaginal mucous membrane is substantially equal to an equivalent impedance value of water, while the minimum value is set to represent a time period of non-ovulation when the detected equivalent impedance on the vaginal mucous membrane is substantially equal to an equivalent impedance value of mammal's blood; and
indicating means (20) for indicating the result of the comparison effected by said comparing means (OP1-OP6).

## Patentansprüche

1. Gerät zur Messung einer optimalen Fruchtbarkeit bei Säugetieren, enthaltend:
eine Einrichtung (10) für die Messung der Natriumionkonzentration zur Bestimmung der Natriumionkonzentration auf der Vaginalschleimhaut eines Säugetieres, wobei die Meßeinrichtung enthält:
eine Meßeinheit (12), die dazu bestimmt ist, in die Vagina des Säugetieres eingeführt zu werden;
mehrere Elektroden (14), die an der Meßeinheit (12) so angeordnet sind, daß sie mit der Vaginalschleimhaut des Säugetieres in Berührung kommen, wenn die Meßeinheit (12) in die Vagina des Säugetieres eingeführt wird;
eine Spannungserzeugungseinrichtung (16) für das Anlegen einer Spannung an die Elektroden (14); und
ein Impedanzmeßgerät (18) für die Messung der Impedanz zwischen den Elektroden (14);
weiterhin gekennzeichnet durch:
Einrichtungen (OP1-OP6), für das Vergleichen des äquivalenten Impedanzpegels, der mit dem Impedanzmeßgerät (18) gemessen wird, mit Bezug auf einen Maximalwert, um einen Zeitraum vor der Ovulation zu messen und anzuzeigen, der für das Einbringen des Spermatozoons in die Vagina optimal ist, und um den gemessenen Wert mit Bezug auf einen Minimalwert zu vergleichen, um einen unfruchtbaren Zeitraum, in dem keine Ovulation auftritt, zu messen und anzuzeigen, wobei der Maximalwert so eingestellt wird, daß er einen Zeitraum kurz vor der Ovulation repräsentiert, wenn die gemessene, äquivalente Impedanz auf der Vaginalschleimhaut im wesentlichen der äquivalenten Impedanz von Wasser gleicht, während ein Minimalwert so eingestellt wird, daß er einen Zeitraum repräsentiert, in dem keine Ovulation auftritt, wenn die gemessene äquivalente Impedanz auf der Vaginalschleimhaut im wesentlichen einem äquivalenten Impedanzwert von Blut des Säugetieres gleicht; und
Anzeigeeinrichtungen (20) für das Anzeigen des Vergleichsergebnisses, das mit den Vergleichseinrichtungen (OP1-OP6) ermittelt wurde.

## Revendications

1. Appareil permettant de détecter la fertilité optimale chez les mammifères, comprenant :
un moyen (10) permettant de mesurer la concentration en ions sodium afin de détecter une concentration en ions sodium mesurée sur la muqueuse vaginale d'un mammifère, ledit moyen de mesure comportant :
une unité de détection (12) qui doit être insérée dans le vagin d'un mammifère ;
une pluralité d'électrodes (14) prévue sur ladite unité de détection (12) de manière à être en contact avec la muqueuse vaginale dudit mammifère lorsque ladite unité de détection (12) est insérée dans le vagin dudit mammifère ;
un moyen générateur de tension (16) permettant d'appliquer une tension aux bornes de ladite pluralité d'électrodes (14) ; et
un détecteur d'impédance (18) permettant de détecter l'impédance entre ladite pluralité d'électrodes (14) ;
caractérisé en ce qu'il comprend, en outre, un moyen (OP1-OP6) permettant de comparer la valeur équivalente d'impédance détectée par ledit détecteur d'impédance (18) à une valeur maximum afin de déterminer et d'indiquer une période de temps, avant l'ovulation, optimale pour injecter des spermatozoïdes dans le vagin, et permettant de comparer ladite valeur détectée à une valeur minimum afin de déterminer et d'indiquer une période de temps d'infertilité lors d'une absence d'ovulation, dans lequel la valeur maximum est fixée de façon à représenter une période de temps précédant immédiatement l'ovulation lorsque l'impédance équivalente détectée sur la muqueuse vaginale est sensiblement égale à une valeur équivalente d'impédance de l'eau, alors que la valeur minimum est fixée de façon à représenter une période de temps d'absence d'ovulation lorsque l'impédance équivalente détectée sur la muqueuse vaginale est sensiblement égale à une valeur équivalente d'impédance du sang du mammifère; et
un moyen indicateur (20) permettant d'indiquer le résultat de la comparaison effectuée par ledit moyen de comparaison (OP1-OP6).
